# EUROPEAN PATENT SPECIFICATION

(11) **EP 0 726 257 B1**
(45) Date of publication and mention of the grant of the patent: **11.04.2001**
(21) Application number: 96101884.3
(22) Date of filing: 09.02.1996
(51) Int. Cl.: C07D 243/02, C07D 491/04, C07D 403/06, C07D 409/06, C07D 405/06, A61K 31/55, C07D 493/04

(54) **1-(Hetero)Arylvinyl-5H-2,3-benzodiazepine derivatives useful for the treatment of central nervous system disorders, and benzopyrylium intermediates for their preparation**
1-(Hetero)Arylvinyl-5H-2,3-benzodiazepinderivative verwendbar zur Behandlung von Erkrankungen des Zentralnervensystems, sowie Benzopyrylium Intermediate zu ihrer Herstellung
Dérivés de 1-(hétéro)arylvinyl-5H-2,3-benzodiazépine utiles dans le traitement des maladies du système nerveux central, et intermédiaires benzopyrylium pour leur préparation

(30) Priority: 09.02.1995 HU 9500385; 24.11.1995 HU 9503353; 09.02.1995 HU 9500386
(43) Date of publication of application: 14.08.1996
(73) Proprietor: EGIS GYOGYSZERGYAR RT., 1106 Budapest (HU)
(72) Inventor: Vágo, Pál, Dr., H-1173 Budapest (HU); Reiter, Jozsef, Dr., H-1022 Budapest (HU); Gyertyán, István, Dr., H-1165 Budapest (HU); Gacsályi, Istvan, H-1021 Budapest (HU); Bilkei-Gorzo, András, Dr., H-1105 Budapest (HU); Egyed, András, Dr., H-1145 Budapest (HU); Andrási, Ferenc, Dr., H-1125 Budapest (HU); Bakonyi, Anna, Dr., H-1138 Budapest (HU); Berzsensyi, Pál, Dr., H-1174 Budapest (HU); Botka, Péter, Deceased (HU); Hámori, Tamás, Dr., H-1031 Budapest (HU); Salamon Haskáné, Cecilia, Dr., H-1068 Budapest (HU); Horváth, Edit, Dr., H-1119 Budapest (HU); Horváth, Katalin, Dr., H-1026 Budapest (HU); Körösi, Jeno, Dr., Deceased (HU); Máté, Györgyné, Dr., H-1046 Budapest (HU); Moravcsik, Imre, H-1095 Budapest (HU); Szentkuti, Eszter, Dr., H-1165 Budapest (HU); Zolyomi, Gábor, Dr., H-1184 Budapest (HU); Blasko, Gábor, Dr., H-1113 Budapest (HU); Daroczi Kazoné, Klára, H-1161 Budapest (HU); Simig, Gyula, Dr., H-1126 Budapest (HU); Tihanyi, Károly, Dr., H-1171 Budapest (HU); Bajnogel, Judit, Dr., H-1151 Budapest (HU)
(74) Representative: Beszédes, Stephan G., Dr.

(56) References cited:
- FR-A- 2 081 426
- FR-A- 2 439 189
- FR-A- 2 501 686
- GB-A- 2 190 677
- CHEMICAL ABSTRACTS, vol. 74, no. 15, 12 April 1971 Columbus, Ohio, US; abstract no. 76293w, G.N. DOROFEENKO ET AL.: "2-Benzopyrylium salts. X. Reaction with nitrogen bases and aromatic aldehydes" page 432; XP002003157 & KHIM. GETEROTSIKL. SOEDIN., no. 10, 1970, pages 1308-12,

## Description

This invention is concerned with new 1-[2'-(substituted) vinyl] -5H-2,3-benzodiazepine derivatives with useful pharmacological effects, particularly on central nervous system disorders, a process and novel intermediates for preparing them as well as medicaments containing these compounds and the use of them.

So far 5H-2,3-benzodiazepine derivatives containing a phenyl, naphthyl, substituted phenyl, furyl, thienyl or hydroxystyryl substituent at the 1 position of the basic molecule skeleton have been described (Hungarian patent specifications Nos. 155,572, 179,018, 195,788, 191,702 and 206,719; J. Chem. Soc. Perkin I. 1973, 2 543; 1980, 1 718; 1984, 849; Il Farmaco-Ed. Sc. 40, 942 [1985]; Chem. Ber. 107, 3 883 [1974]). A group of the known compounds has influences on the central nervous system, the 5H-2,3-benzodiazepine derivatives belonging to the other group possess a positive inotropic effect and do not exert any central nervous activity.

Thus in FR-A-2,439,189 there have been described 5H-2,3--benzodiazepine derivatives containing in the 1 position a hydrogen atom or a C₁₋₅-alkyl group or a dialkylaminoalkyl, amino, alkylamino, dialkylamino or styryl group or a phenyl group substituted by a C₁₋₃-alkyl group or an alkyl group, optionally bearing 1 to 3 substituents, such as halogen atoms, hydroxy, acyloxy, methyl, trifluoromethyl, nitro, amino, methylenedioxy, C₁₋₄-alkoxy and/or aralkoxy groups or containing in the 1 position a heterocyclic group having 1 or 2 nitrogen, oxygen and/or sulphur atoms, having in the 4 position a hydrogen atom or a C₁₋₄-alkyl group, hydroxymethyl, formyl, carboxy, carbalkoxy, aryl or heterocyclic group, containing in the 5 position a hydrogen atom or a C₁₋₄-alkyl group or dialkylaminoalkyl, alkylamino, dialkylamino or aryl group and containing in the 7 and 8 positions a hydrogen or halogen atom or a nitro, amino, hydroxy, acyloxy, C₁₋₃-alkyl, C₁₋₅-alkoxy, dialkylaminoalkoxy or aralkoxy group or the 7 and 8 positions are bridged by a methylenedioxy group or by a carboxylic acid rest. No specific compound having a styryl group in the 1 position has been mentioned. For these compounds spontaneous motor activity inhibiting (sedative), analgesic and narcose potentiating effects have been indicated as activities influencing the central nervous system but no further effects on the central nervous system.

Furthermore from FR-A-2,501,686 there have been known 3,4--di-[hydro]-5H-2,3-benzodiazepine derivatives containing in the 1 position a phenyl substituent, optionally substituted by 1 or 2 substituents from the group of halogen atoms or hydroxy, C₁₋₄-alkoxy or benzyloxy groups, or containing in the 1 position a furyl or thienyl group, having a hydrogen atom or a methyl group in the 4 position, a hydrogen atom or a C₁₋₄-alkyl group in the 5 position and hydrogen atoms or C₁₋₄-alkoxy, C₄₋₇-cycloalkoxy or benzyloxy groups in the 6 and 7 positions. For these compounds the same indications have been indicated as for those of FR-A-2,439,189. According to this publication the said compounds can be prepared from the corresponding compounds having a double bond between the 3 and 4 positions by reducing the latter with metal hydrides or organic and/or complex metal hydrides.

Moreover from GB-A-2,190,677 there have been known 1-(hydroxystyryl) -5H-2,3-benzodiazepine derivatives containing in the 1 position a hydroxystyryl group the phenyl group of which is optionally substituted by a halogen atom or a C₁₋₄-alkoxy group, having a methyl group in the 4 position, containing a hydrogen atom or a C₁₋₄-alkyl group in the 5 position and having C₁₋₄-alkoxy groups in the 7 and 8 positions or the 7 and 8 positions are bridged by a methylenedioxy group. For these compounds positive inotropic (cardiotonic) effects have been indicated as pharmaceutical indications.

Furthermore in FR-A-2,081,426 there have been described 2,3-di- [hydro] -benz[f] -1,4-oxazepine derivatives which can have in the 2, 3, 4 and 5 positions lower alkyl, lower alkenyl or lower alkinyl groups or aryl groups, optionally substituted by aralkyl, aryloxyalkyl or arylthioalkyl groups, optionally substituted on the aromatic ring, possibly alternatively the 5 position being substituted by a pyridyl or pyrimidyl group attached directly or by means of a C₁₋₃-alkyl chain and the carbon atoms of the 2 and 3 being bridged by an alkyl chain thus forming a cyclic ring having 5, 6 or 7 carbon atoms, and also the benzene ring of these 2,3-di-[hydro]-benz[f]-1,4-oxazepines optionally having substituents. In Example 32 the preparation of 5-(4-chlorostyryl)-2,3-dihydro-8-methoxy-3-methylbenz[f]-1,4-oxazepine has been described. For the compounds of this publication antispasmodic effects in the gastro-intestinal and urinary tracts, the biliary ducts, in bronchial constrictions and in the stage of coronary and cerebral vascular spasms have been indicated; for some compounds but not for the said compound of Example 32 effects on the central nervous system, namely cataleptic and ptotic activities have been indicated.

In CHEMICAL ABSTRACTS, vol. 74, no. 15, 12 April 1971 there has been described a process for preparing 1-[styryl]-3-- [methyl] -6,7-di- [methoxy] -2-benzopyrilium perchlorate and 1- [styryl] -3- [methyl] -6,7- [methylenedioxy] -2-benzopyrilium perchlorate, respectively, by reacting 1,3-di-[methyl]-6,7--di-[methoxy]-2-benzopyrilium perchlorate or 1,3-di-[methyl]--6,7-[methylenedioxy]-2-benzopyrilium perchlorate, respectively, with toluene.

The known 2,3-benzodiazepines having effects on the central nervous system possess anxiolytic and antiaggressive activities, but - contrary to the traditional 1,4-benzodiazepines - they do not exert a muscle relaxant effect, and furthermore the anxiolytic effect is complemented by a neuroleptic activity. The known 1-(4'-<amino>-phenyl)-4-- (methyl) -7,8-di- (methoxy) -5H-2,3-benzodiazepine [nerisopam] belonging to this group also exerts both anxiolytic and neuroleptic activities, but it has a considerable cataleptogenic side-effect, too.

Besides, about 20% of the civilized world's population suffer from anxiety, and thus the treatment of this disease is of high importance. The overwhelming majority of the drugs applied in the therapy of anxiety are 1,4-benzodiazepine derivatives. However, said drugs exert some undesirable side-effects, such as sedation, muscle relaxation and somnolence (Patel, J. B. and Malich, J. B.: Neuropharmacological profile of an anxiolytic, in: Anxiolytics: Neurochemical behavioural and clinical perspectives. {Eds.: Malich, J. B., Enna S. J. and Yamuuamura, H. I., Raven Press, New York 1983, pp. 173 to 191}; File, S. L.: The contribution of behavioural studies to the neuropharmacology of anxiety, Neuropharmacology 1987, 26, 877 to 866).

Hence the problem underlying to the invention is to create novel 1-[2'-(substituted) vinyl] -5H-2,3-benzodiazepine derivatives showing valuable pharmacological properties, particularly for the treatment of central nervous system disorders with long duration of action, above all anxiolytic activity nearly as strong as that the known 1,4- and 2,3-benzodiazepines, and among these such being outstanding even at low doses, but - contrary to the known anxiolytics - having practically no influence on the movement activity even at high doses, and/or neuroleptic activity comparable to that of 1- (4'-<amino>-phenyl) -4- (methyl) -7, 8-di- (methoxy) -5H-2,3--benzodiazepine [nerisopam], but devoid of any cataleptic side-effect hindering the therapeutic application, a process and intermediates for preparing them as well as medicaments containing these compounds and the use of them.

Surprisingly this has been solved by the invention.

Thus a subject-matter of the invention are 1-[2'-- (substituted) vinyl] -5H-2,3-benzodiazepine derivatives of general formula 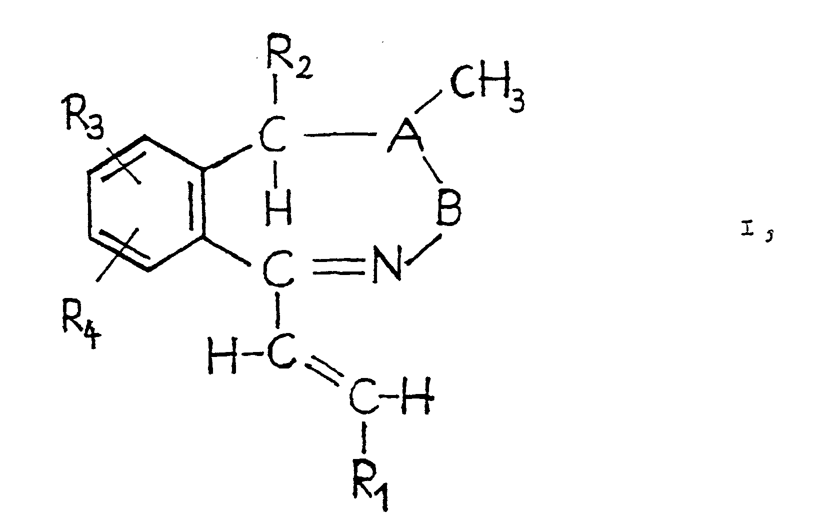 wherein
- A and B: together form a group of formula 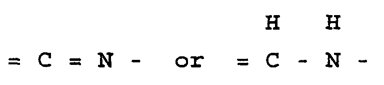
- R₁: represents phenyl, carrying 1 to 3 identical or different substituent(s) selected from the group consisting of halogen, trifluormethyl or cyano,
- R₂: stands for hydrogen and
- R₃ and R₄: together form a 7,8- or 8,9-methylenedioxy group
as well as acid addition salts and stereoisomers and mixtures thereof.

It is preferred that the halogen atom(s) by which the phenyl group by which R₁ may be represented may be substituted is/are fluorine, bromine and/or chlorine, particularly fluorine or bromine, most particularly fluorine.

In case R₁ stands for phenyl carrying more than 1 substituted preferred groups are 2,4-di-(chloro)-phenyl, 3,4-di--(chloro)-phenyl and 2,6-di- (chloro) -phenyl.

A preferred group of 1-[2'-(substituted) vinyl]-5H-2,3--benzodiazepine derivatives according to the invention are those in which
- A and B: together form a group of formula

= C = N - ,
- R₁: represents phenyl, carrying 1 or 2 fluoro, cyano and/or trifluoromethyl substituent(s),
- R₂: stands for hydrogen and
- R₃ and R₄: together form a 7,8-methylenedioxy group,
as well as acid addition salts and stereoisomers and mixtures thereof.

Particularly preferred 1- [2' - (substituted) vinyl]-5H-2,3--benzodiazepine derivatives according to the invention are the following ones: 1- [4'- (fluoro) -styryl] -4- [methyl] -7,8--[methylenedioxy]-5H-2,3-benzodiazepine as well as acid addition salts and stereoisomers and mixtures thereof.

Suitably the acid addition salts of the 1-[2'-(substituted) vinyl] -5H-2,3-benzodiazepine derivatives of formula I according to the invention are pharmaceutically acceptable ones. Advantageous acid addition salts of the 1-[2'-(substituted) vinyl] -5H-2,3-benzodiazepine derivatives of formula I according to the invention are those with inorganic acids, e.g. hydrogenhalides, such as hydrochloric acid or hydrobromic acid, sulfuric acid, phosphoric acid or perhaloacids, such as perchloric acid, organic carboxylic acids, e.g. fumaric, acetic, propionic, glycolic, maleic, hydroxymaleic, ascorbinic, citric, malic, salicylic, lactic, cinnamic, benzoic, phenylacetic, p-aminobenzoic, p-hydroxybenzoic or p-aminosalicyclic acid, alkylsulfonic acids, e.g. methanesulfonic or ethanesulfonic acid, or arylsulfonic acids, e.g. p-toluenesulfonic, p-bromophenylsulfonic, naphthylsulfonic or sulfanilic acid.

According to a further aspect of the present invention there is provided for a process for preparing the 1-[2'-- (substituted) vinyl]-5H-2,3-benzodiazepine derivatives according to the invention, which is characterized in that
a) in a manner known per se for preparing 1-[2'-(substituted) vinyl] -5H-2,3-benzodiazepine derivatives of general formula I, wherein A and B together form a group of formula = C = N - and R₁, R₂, R₃ and R₄ are as above defined, reacting 2-benzopyrilium perchlorate derivatives of general formula 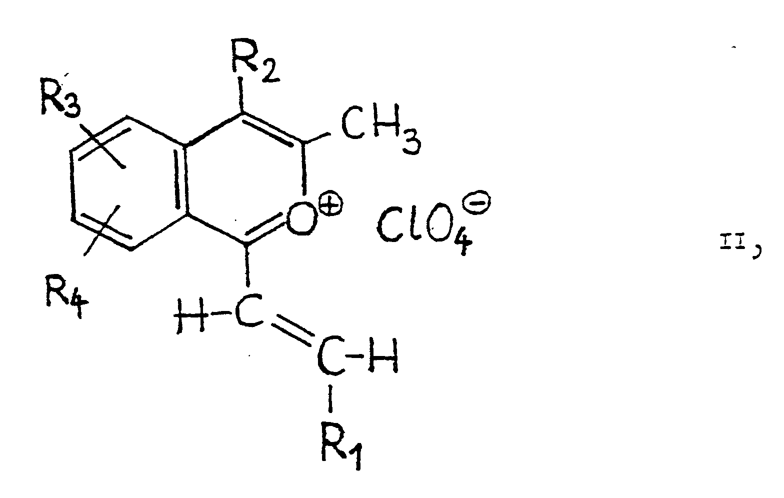 wherein R₁, R₂, R₃ and R₄ are as above defined, with hydrazine hydrate or
b) for preparing 1- [2'-(substituted) vinyl]-5H-2,3--benzodiazepine derivatives of general formula I, wherein A and B together form a group of formula 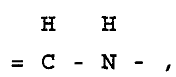 R₁ stands for phenyl carrying a fluoro atom, a trifluoromethyl or a cyano group, R₂ represents hydrogen and R₃ and R₄ together form a 7,8- or 8,9-methylenedioxy group, in a manner known per se reducing 1-[2'-(substituted) vinyl] -5H-2,3--benzodiazepine derivatives of general formula I, wherein A and B together form a group of formula = C = N - and R₁, R₂, R₃ and R₄ are as above defined, with complex metal hydrides, boranes and/or borane complexes,
and optionally in a manner known per se converting the 1-[2'-- (substituted) vinyl] -5H-2,3-benzodiazepine derivatives of general formula I obtained by any of the above variants a) or b) into acid addition salts thereof and/or optionally converting acid addition salts of 1-[2'-(substituted) vinyl]--5H-2,3-benzodiazepine derivatives of general formula I obtained by any of the above variants a) or b) into the corresponding free 1- [2' - (substituted) vinyl]-5H-2,3--benzodiazepine derivatives of general formula I and/or into other acid addition salts and/or resolving stereoisomer mixtures of the 1- [2' - (substituted) vinyl]-5H-2,3-benzodiazepine derivatives of general formula I or acid addition salts thereof into their stereoisomers or converting the stereoisomers into mixtures thereof.

The reaction of the 2-benzopyrilium perchlorate derivatives of general formula II with hydrazine hydrate according to variant a) of the process according to the invention is preferably carried out in 1 or more solvent(s), but it also can be performed without using any solvent, in the excess of the applied hydrazine hydrate. As [a] solvent(s) polar and/or apolar solvent(s), particularly water, C₁₋₄-alcohol(s), dioxane, tetrahydrofurane, dichloromethane chloroform , dimethylformamide, dimethyl sulfoxide or pyridine or mixtures thereof may be used. Suitably the reaction is carried out at a temperature of from 0°C to the boiling point of the reaction mixture, preferably from +10°C to +120°C. It is preferable to carry out the reaction with concentrated (90 to 100% by weight) hydrazine hydrate used in an excess of 1 to 3 mole(s).

According to a preferred embodiment of variant a) of the process according to the present invention the 2-benzopyrilium perchlorate derivatives of general formula II are reacted with 3 molar equivalents of 90 to 100% by weight hydrazine hydrate in a C₁₋₄-alcohol, particularly ethanol, at room temperature, the crude product is separated from the reaction mixture, the salt-like side-products are dissolved from the desired product in hot water and the latter product is filtered off and optionally either suspended in or recrystallized from a suitable solvent, particularly a C₁₋₄-alcohol.

According to another preferred embodiment of variant a) of the process according to the present invention the 2-benzopyrilium perchlorate derivatives of general formula II are suspended in a solvent and reacted with 3 equivalents of 90 to 100% by weight of hydrazine hydrate first at room temperature for 1 to 3 hours, then either at a temperature of from 45°C to 50°C for 10 to 50 minutes or at a temperature of from 70°C to 100°C for half an hour. When the 2-benzopyrilium perchlorate derivative of general formula II is scarcely soluble at room temperature or when the 1-[2'-(substituted) vinyl]-5H-2,3-benzodiazepine derivative of general formula I begins to separate from the reaction mixture during the reaction, it is preferable to apply an elevated temperature at the end of the reaction.

According to a further preferred embodiment of variant a) of the process according to the invention the 2-benzopyrilium perchlorate derivatives of general formula II are added to a mixture of 3 equivalents of 90 to 100% by weight hydrazine hydrate and dimethylformamide at a temperature of from 10°C to 15°C , and the reaction mixture is kept at room temperature. The end-product separates from the solution upon adding some water to the mixture. The side-product is then washed out of it with water and the end-product is optionally purified by recrystallization or boiling in an alcohol.

For the reduction of the 1-[2'-(substituted) vinyl]-5H--2,3-benzodiazepine derivatives of general formula I, wherein A and B together form a group of the formula = C = N - , R₁ represents phenyl carrying a fluoro atom, a trifluoromethyl or a cyano group, R₂ stands for hydrogen and R₃ and R₄ together form 7,8- or 8,9-methylenedioxy group, with complex metal hydrides, boranes and/or borane complexes according to variant b) of the process according to the invention suitably the following reducing agents may be applied: sodium borohydride, lithium aluminium hydride, boranes and borane complexes. The reductuon is preferably carried out in 1 or more solvent(s). For this purpose advantageously water, 1 or more C₁₋₄-alcohol(s), C₁₋₄-carboxylic acid(s), solvent(s) of ether type, aromatic hydrocarbon(s), chlorinated aliphatic hydrocarbon(s) or pyridine or mixtures thereof is/are used. The solvents or solvent mixtures applicable in a given case depend on the applied reducing agent.

Suitably the reduction is carried out at a temperature of from 0°C to 100°C using preferably 1.1 to 25 molar equivalent(s) of reducing agent.

According to an embodiment of variant b) of the process according to the invention the starting 1-[2'-(substituted) vinyl]-5H-2,3-benzodiazepine derivatives of general formula I, wherein A and B together form a group of formula = C = N - , are dissolved or suspended in methanol, an excess of concentrated hydrochloric or acetic acid is added thereto, and sodium borohydride is added to the thus obtained hydrochloride or acetate. After working up the reaction mixture the desired 3,4-dihydro compound, i.e. 1-[2'-(substituted) vinyl]-5H-2,3-benzodiazepine derivative of general formula I, wherein A and B together form a group of formula 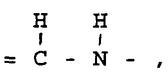 is obtained by crystallization.

According to a preferred embodiment of variant b) of the process according to the invention 1.5 to 2.0 equivalents of borotrifluoride etherate are added to solutions or suspensions of the starting 1-[2'-(substituted) vinyl] -5H-2,3-benzodiazepine derivatives of general formula I, wherein A and B together form a group of formula = C = N - , in dry dichloromethane, preferably at a temperature of from 10°C to 15°C, to the solution of the thus obtained complex 1.1 equivalent of borane-trimethylamine complex, for example, compound No. 17,898-5 of the Aldrich Catalogue, is added, and the reaction mixture is stirred, preferably at 25°C for 0.5 to 4 hour(s). The organic phase is then treated with sodium carbonate, washed with water, dried, evaporated, the desired product is crystallized, filtered and optionally recrystallized from an appropriate solvent, e.g. from a C₁₋₄-alcohol, or suspended in an appropriate solvent.

According to a still further preferred embodiment of variant b) of the process according to the invention the 1-[2'-(substituted) vinyl] -5H-2,3-benzo-diazepine derivatives of general formula I, wherein A and B together form a group of formula = C = N - , are dissolved or suspended in dry tetrahydrofurane, cooled to a temperature of from 0°C to 5°C, 1 molar equivalent of lithium aluminium hydride is added thereto and the reaction mixture is stirred at room temperature for 2 hours. The complex is then decomposed and the organic phase is evaporated. The evaporation residue is subjected to column chromatography or crystallization in order to obtain the desired 1-[2'-(substituted) vinyl] -5H-2,3-benzodiazepine derivative of general formula I, wherein A and B together form a group of formula

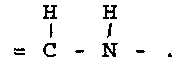

The starting 2-benzopyrilium perchlorate derivatives of general formula II can be prepared in a manner analogous to the known synthesis methods provided, e.g. in Khim. Geterotsikl. Soedin. 1970, 1 308 [C.A. 74, 7 629 w (1971)]; 1973, 568, 1 458 [C.A. 79, 18 629 c (1973), 80, 70 649 u (1974)].

As already mentioned, the 1-[2'-(substituted) vinyl]-5H--2,3-benzodiazepine derivatives according to the invention possess valuable pharmaceutical properties, particularly central nervous activities, most particularly anxiolytic, antipsychotic and antiaggressive effects, at the same time being devoid of any cataleptogenic side-effect.

Hence by the invention also there are provided for medicaments which are characterized in that they contain 1 or more 1-[2'-(substituted) vinyl]-5H-2,3-benzodiazepine derivative(s) according to the invention as [an] active ingredient(s), advantageously in admixture with 1 or more inert solid and/or liquid pharmaceutical carrier(s) and/or auxiliari(es).

The medicaments according to the invention can be in the form of pharmaceutical preparations. These can be prepared by methods known per se by admixing the 1 or more 1-[2'-(substituted) vinyl]-5H-2,3-benzodiazepine derivative(s ) with 1 or more inert solid and/or liquid carrier(s) and/or auxiliari(es) and bringing the mixture to a galenic form.

The pharmaceutical preparations of the present invention may be in forms suitable for oral administration, e.g. tablets, pills, coated pills, dragées, solid or soft gelatin capsules, solutions, emulsions or suspensions, for parenteral administration, e.g. injection solutions, or for rectal administration, e.g. suppositories.

Tablets, coated tablets, dragees and solid gelatin capsules can contain as carrier(s) e.g. lactose, corn starch, potato starch, talc, magnesium carbonate, magnesium stearate, calcium carbonate and/or stearic acid and/or [a] salt(s) thereof. Soft gelatin capsules can contain as carrier(s) e.g. [a] vegetable oil(s), fat(s), wax(es) and/or polyol(s) of suitable consistency. Solutions and syrups can contain as carrier(s) e.g. water, [a] polyol(s), such as polyethylene glycol, saccharose and/or glucose. The injection solutions can contain e.g. water, [an] alcohol(s), polyol(s), glycerol and/or [a] vegetable oil(s) as carrier(s). The suppositories can contain as carrier(s) e.g. [an] oil(s), wax(es), fat(s) and/or polyol(s) of suitable consistency.

In addition, the pharmaceutical preparations according to the invention may contain 1 or more auxiliari(es) usually applied in the pharmaceutical technique, e.g. [a] wetting agent(s), sweetening agent(s), aroma substance(s), salt(s) causing the change of osmotic pressure, and/or buffer(s). They may further contain 1 or more other active ingredient(s), too.

The daily dose of the 1-[2'-(substituted) vinyl]-5H-2,3--benzodiazepine derivatives according to the invention can vary within wide ranges depending on several factors, e.g. on the activity of the active ingredient, the patient's condition and age and the severity of the disease. The preferred oral dose is generally 0.1 to 500 mg/day. It has to be stressed that the above dose is only of informative character and the administered dose must always be determined by the physician therapeutist.

According to a further aspect of the present invention there is provided for the use of the 1-[2'-(substituted) vinyl]-5H-2,3-benzodiazepine derivatives according to the invention for preparing medicaments, particularly for the treatment of central nervous system disorders.

The 1-[2'-(substituted) vinyl] -5H-2,3-benzodiazepine derivatives according to the present invention are to be administered to the patients in effective amounts.

Surprisingly, 1-[2'-(substituted) vinyl] -5H-2,3-benzodiazepine derivatives of general formula I, particularly those, wherein R₁ stands for phenyl carrying a fluoro atom, a trifluoromethyl or a cyano group, R₂ represents hydrogen and R₃ and R₄ together form a 7,8- or 8,9-methylenedioxy group, and their acid addition salts possess outstanding anxiolytic activity, and at the same time they are practically devoid of any sedative effect. This characteristic is highly advantageous in the therapy of anxiety disorders. These molecules do not bind to the homophthalazine binding site.

The activity of the 1-[2'-(substituted) vinyl]-5H-2,3--benzodiazepine derivatives according to the invention has been proved by the following experiments.

The cataleptic effect of the compounds was measured both in mice (Schlichtegroll: Arzneim. Forsch., 8, 489, [1958]) and in rats (Morpurgo: Arch. Int. Pharmacodyn., 137, 87, [1962]). The results are shown in Table I.

**Table I**

| Compound (No. of Example) | Catalepsy in mice Minimal effective dose [MED] mg/kg p.o. | Catalepsy in rats Minimal effective dose [MED] mg/kg p.o. |
|---|---|---|
| 1 | >100 | >100 |
| 4 | >100 | >100 |
| 1-(4'-<amino>-phenyl) -4-(methyl) -7,8-di-(methoxy) -5H-2,3-benzodiazepine [nerisopam] {reference substance B} | 40 | 60 |
| 2-(chloro)-10-(3'-<dimethylamino>-propyl)-phenothiazine [chlorpromazine] {CPZ} <reference substance D> | 10 | 20 |

From the test results of above Table I it can be taken that the 1- [2' - (substituted) vinyl]-5H-2,3-benzodiazepine derivatives according to the invention do not possess cataleptic activity, which would limit their therapeutic application. This fact represents a significant advantage over 2- (chloro)-10-(3'-<dimethylamino>-propyl) -phenothiazine [chlorpromazine] {CPZ} <reference substance D> and and 1-(4'-<amino>-phenyl) -4- (methyl) -7,8-di- (methoxy) -5H-2,3--benzodiazepine [nerisopam] {reference substance B} possessing a cataleptic side-effect.

As it has been mentioned, 1-[2'-(substituted) vinyl]-5H--2,3-benzodiazepine derivatives according to the invention besides outstanding anxiolytic activities - in contrast to the known 1,4- and 2,3-benzodiazepines - are practically devoid of any sedative effect. This characteristic is advantageous from the point of view of the therapeutic application. Besides, these compounds exert a weak anticonvulsive activity.

The anxiolytic activity of these compounds was investigated in the elevated plus maze test. Male rats belonging to the Sprague Dawley strain and weighing 220 to 260 g were used as test animals. The test was carried out with the aid of a wooden plus-shaped maze elevated to a height of 50 cm and containing two enclosed and two open arms (50 cm long and 15 cm wide). After 60 minutes pretreatment the animals were placed into the maze and observed for 5 minutes. The drug effect was expressed as percent increase of the time spent on the open arms and number of open arm entries. The minimum effective dose (MED) which caused a significant increase of the time spent on the open arms was calculated for every substance [Pelow, S., Chopin, P., File, S.E., Briley, M.: J. Neurosci. Methods 14, 149 to 167 [1985]). The data are shown in the following Table II.

**Table II**

| Minimum effective dose | |
|---|---|
| Compound (No. of Example) | Dose mg/kg p.o. |
| 1 | 0.003 |
| 2 | 0.1 |
| 4 | 0.3 |
| 7 | 1 |
| 5 | 0.3 |
| 7- (chloro)-2,3-di-(hydro)-1-(methyl) -5-(phenyl) -1H-1,4-benzodiazepin-2-(one) [diazepam] {reference substance A} | 1 |
| 1-[3'-(chloro)-phenyl] -4- [methyl] -7,8-di [methoxyl - 5H-2,3-benzodiazepine [girisopam] {reference substance B} | 13 |

From the above table II it can be seen that the tested 1-[2'-(substituted) vinyl]-5H-2,3-benzodiazepine derivatives according to the invention possess an anxiolytic activity surpassing that of 7-(chloro)-2,3-di-(hydro)-1-(methyl)-5-- (phenyl)-1H-1,4-benzodiazepin-2- (one) [diazepam] {reference substance A} and 1- [3' - (chloro) -phenyl] -4- [methyl] -7,8--di [methoxy] -5H-2,3-benzodiazepine [girisopam] {reference substance B}. The activity of the most effective compound according to the invention is highly superior to the said reference substances.

The effect of the 1-[2'-(substituted) vinyl]-5H-2,3--benzodiazepine derivatives according to the invention on the spontaneous motor activity was tested according to the method of Borsy et al. Groups consisting of 3 mice were treated orally with different doses of the compounds to be tested. Then the animals were placed into a 10-channel Dews system equipment, wherein the number of interruptions if infrared beam within 30 minutes was counted. From these data 50% inhibiting doses (ID₅₀) were determined with the aid of linear regression (Borsy, J., Csányi, E., Lázár, I.: Arch. Int. Pharmacodyn. 124, 1 [1960]). The data are shown in Table III.

**Table III**

| Compounds (No. of Example) | ID₅₀ mg/kg p.o. |
|---|---|
| 1 | >100 |
| 2 | >100 |
| 3 | >100 |
| 6 | >100 |
| 7-(chloro)-2,3-di-(hydro)-1-(methyl)-5-(phenyl)-1H-1,4-benzodiazepin-2-(one) [diazepam] {reference substance A} | 23 |

From the data of the above Table III it can be taken that when administered in oral doses of 100 mg/kg the tested 1-[2'-(substituted) vinyl] -5H-2,3-benzodiazepine derivatives according to the invention practically do not have influence on the spontaneous motor activity of mice (differences within ±12% were observed), contrary to the said reference substance having an oral ID₅₀ value of 23 mg/kg. Consequently, considering the sedative activity 1-[2'-(substituted) vinyl]-5H-2,3-benzodiazepine derivatives according to the invention are much more favourable than 7-(chloro)-2,3-di(hydro) -1-(methyl)-5-(phenyl) -1H-1,4-benzodiazepin-2-(one) [diazepam] {reference substance A}.

The inhibiting effect against convulsions caused by 6,7,8,9-tetrahydro-5H-tetrazoloazepine [pentetrazole] was defined by the modified method of Benziger and Hane. Groups of male and female mice belonging to the NMRI strain and weighing 20 to 25 g were used. The tonic extensor convulsions of the hind limbs caused by intraperitoneally administered 125 mg/kg of 6,7,8,9-tetrahydro-5H-tetrazoloazepine [pentetrazole] were registered on the animals of groups consisting of 6 to 12 animals. The test compounds and the carrier were administered 1 hour before administering the 6,7,8,9-tetrahydro-5H-tetrazoloazepine [pentetrazole] (Benziger, R., Hane, D.: Arch. Int. Pharmacodyn. 167, 245 [1967]). The results are given in the following Table IV.

**Table IV**

| Compound (No. of Example) | Effect 100 mg/kg p.o. |
|---|---|
| 5 | - 57% |
| 3 | - 44% |
| 6 | - 44% |
| 5H-dibenz [b,f] azepine-5-carboxamide [carbamazepine] {reference substance C} | ID₅₀ = 7.1 mg/kg p.o. |

According to the test results of the above Table IV the tested 1-[2'-(substituted) vinyl]-5H-2,3-benzodiazepine derivatives according to the invention possess a mild anticonvulsive activity.

Those starting 2-benzopyrilium perchlorate derivatives of general formula II, wherein R₁ stands for phenyl carrying a fluoro atom, a trifluoromethyl or a cyano group, R₂ represents hydrogen and R₃ and R₄ together form a 6,7- or 7,8-methylendioxy group, so far have not been described in the literature.

Hence a subject-matter of the invention are also 2-benzopyrilium perchlorate derivatives of general formula 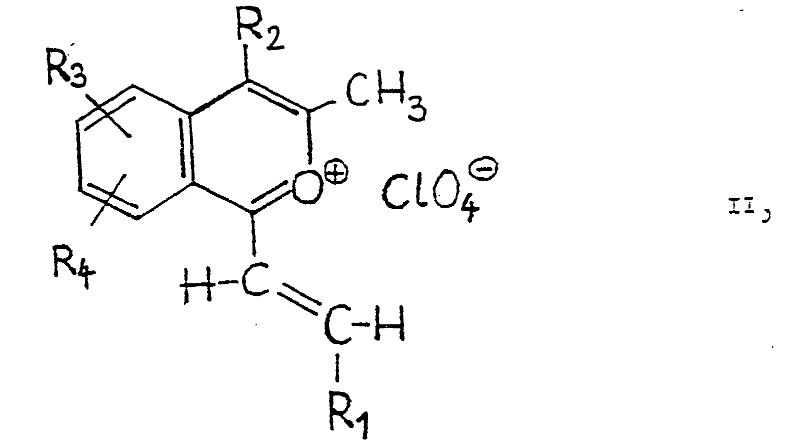 wherein
- R₁: stands for phenyl carrying a fluoro atom, a trifluoromethyl or a cyano group,
- R₂: represents hydrogen and
- R₃ and R₄: together form a 6,7- or 7,8-methylenedioxy group.

These 2-benzopyrilium perchlorate derivatives of general formula II are the cause of the surperior pharmacological effects of the end products 1-[2'-(substituted) vinyl]-5H-2,3--benzodiazepine derivatives of formula I and of their acid addition salts according to the invention preparable from them.

The invention is further illustrated by means of the following Examples.

### Example 1

### 1-[4'-(Fluoro)-styryl]-4-[methyl]-7,8-[methylenedioxy]-5H--2,3-benzodiazepine

To a suspension of 15.1 g (37 mmoles) of powdered 1-[4'-- (fluoro) -styryl] -3- [methyl] -6,7-[methylenedioxy]-2--benzopyrilium perchlorate in 150 ml of methanol 4.1 ml (82 mmoles) of hydrazine hydrate are added, and the mixture is stirred until complete dissolution. Then it is allowed to stand for 1 hour at 25°C, evaporated in vacuo, the residue is stirred in the mixture of 50 ml of water and 50 ml of ethyl acetate. The ethyl acetate phase is separated, dried over magnesium sulfate and the solvent is evaporated in vacuo. The residue is dissolved in 50 ml of 99.5% by volume ethanol and the end-product is separated from the ethanol solution by adding some water to it. Thus 8.9 g of crude product are obtained, which is recrystallized from 99.5% by volume ethanol. Thus 8.1 g (68%) of substance pure on TLC are obtained.
M.p.: 141 to 144°C.

### Example 2

### 1-[4'-(Trifluoromethyl)-styryl]-4-[methyl]-7,8-[methylenedioxy]-5H-2,3-benzodiazepine

To a suspension of 9.17 g (20 mmoles) of powdered 1-[4'-(trifluoromethyl)-styryl]-3-[methyl]-6,7-[methylenedioxy]--2-benzopyrilium perchlorate in 100 ml of methanol 2.5 ml (50 mmoles) of hydrazine hydrate are added, and the reaction is carried out as specified in Example 1. Thus 6.7 g of crude product are obtained, which is purified by recrystallization from 99.5% by volume ethanol. Thus 6.1 g (82%) of the desired substance pure on TLC are obtained.
M.p.: 188 to 191°C.

### Example 3

### 1-[4'-(Cyano)-styryl]-4-[methyl]-7,8-[methylenedioxy]-5H--2,3-benzodiazepine

To a suspension of 9.30 g (23.1 mmoles) of 1-[4'-(cyano)--styryl]-3-[methyl]-6,7-[methylenedioxy]-2-benzopyrilium perchlorate prepared according to Example 10 in 100 ml of methanol 3.5 ml (70 mmoles) of hydrazine hydrate are dropped, under stirring. The reaction mixture is stirred for 3 hours at room temperature, filtered and some water is added to the filtrate under stirring. The separated crystals are filtered off and recrystallized from ethanol. Thus 2.90 g (38%) of the desired compound are obtained.
M.p.: 197.5 to 202.5°C.

### Example 4

### 1-[3'-(Fluoro)-styryl]-4-[methyl]-7,8-[methylenedioxy]-5H-- 2,3-benzodiazepine

8.17 g (0.02 mole) of 1-[3'-(fluoro)-styryl]-3-[methyl]--6,7- [methylenedioxy] -2-benzopyrilium perchlorate prepared according to Example 11 are suspended in 100 ml of methanol and 2.5 ml (0.05 mole) of hydrazine hydrate are dropped to the suspension. Then the reaction mixture is stirred at room temperature for 2 hours, filtered and the filtrate is evaporated. The residue is dissolved in the mixture of 50 ml of water and 50 ml of ethyl acetate, the organic phase is separated, dried over magnesium sulfate and evaporated again. The residue is crystallized from 99.5% by volume ethanol and recrystallized from acetonitrile. Thus 4.30 g (67%) of the desired product are obtained, which is pure on TLC. M.p.: 161 to 162°C.

### Example 5

### 1-[2'-(Fluoro)-styryl)-4-[methyl]-7,8-[methylenedioxy]-5H--2,3-benzodiazepine

8.17 g (0.02 mole) of 1-[2'-(fluoro)-styryl]-3-[methyl]--6,7- [methylenedioxy] -2-benzopyrilium perchlorate prepared according to Example 12 are stirred in 100 ml of acetonitrile, and 2.5 ml (0.05 mole) of hydrazine hydrate are dropped to the mixture. It is stirred further for 2 hours at room temperature, filtered and the filtrate is evaporated. The evaporation residue is dissolved in the mixture of 50 ml of water and 50 ml of ethyl acetate, the organic phase is separated, dried over magnesium sulfate and evaporated again. The residue is crystallized from ethanol and purified by recrystallization from 99.5% by volume ethanol. Thus 3.79 g (59%) of the desired compound are obtained, which is pure on TLC and melts at 138 to 141°C.

### Example 6

### 1-[4'-(Fluoro)-styryl]-4-[methyl]-8,9-[methylenedioxy]-5H--2,3-benzodiazepine

2.70 g (6.6 mmoles) of 1-[4'-(fluoro)-styryl]-3-[methyl]---7,8- [methylenedioxy] -2-benzopyrilium perchlorate prepared according to Example 13 are stirred in 50 ml of acetonitrile, and 0.80 ml (16.0 mmoles) of hydrazine hydrate are dropped to the mixture. Then it is stirred further for 2 hours at room temperature, filtered and the filtrate is evaporated. The evaporation residue is dissolved in the mixture of 10 ml of water and 10 ml of ethyl acetate. The organic phase is separated, dried over magnesium sulfate and evaporated again. The product is obtained from the evaporation residue by purification on a Kieselgel 60 column. Thus 1.10 g (52%) of the desired substance pure on TLC is obtained.

### Example 7

### 1-[4'-(Fluoro)-styryl]-4-[methyl]-7,8-[methylenedioxy]--3,4-di-[hydro]-5H-2,3-benzodiazepine

To a mixture of 2.0 g (6.2 mmoles) of 1-[4'-(fluoro)--styryl]-4-[methyl]-7,8-[methylenedioxy]-5H-2,3-benzodiazepine prepared according to Example 1 and 10 ml of glacial acetic acid a solution of 3.0 g (79 mmoles) of sodium borohydride in 10 ml of water is added in small portions, while the temperature of the reaction mixture is kept below 35°C. The mixture is then stirred for further 2 hours and made alkaline by adding some sodium carbonate solution to it. The alkaline solution is extracted three times with 20 ml of ethyl acetate. The ethyl acetate phases are dried, evaporated and the evaporation residue is crystallized from 99.5% by volume ethanol. The crude product is purified by recrystallization from 99.5% by volume ethanol. Thus 1.39 g (69%) of substance pure on TLC is obtained.
M.p.: 170 to 174°C.

Preparation of the new starting 2-benzopyrilium perchlorate derivatives:

### Example 8

### 1-[4'-(Fluoro)-styryl]-3-[methyl]-6,7-[methylenedioxy]--2-benzopyrilium perchlorate

A mixture of 15.10 g (0.05 mole) of 1,3-di-[methyl]-6,7-- [methylenedioxy]-2-benzopyrilium perchlorate, 80 ml of glacial acetic acid and 6.20 g (0.05 mole) of 4-[fluoro]-benzaldehyde is stirred on an oil bath of 150°C for 3 hours. The reaction mixture is cooled to 80°C, filtered, the precipitate is washed twice with 10 ml each of acetic acid and twice with 30 ml each of ethyl acetate and dried at room temperature. Thus 9.60 g (47%) of the desired product decomposing at about 203 to 204°C are obtained. The substance can be used for the further reactions without any purification.

### Example 9

### 1-[4'-(Trifluoromethyl)-styryl]-3-[methyl]-6,7--[methylenedioxy]-2-benzopyrilium perchlorate

A mixture of 15.10 g (0.05 mole) of 1,3-di-[methyl] -6,7--[methylenedioxy]-2-benzopyrilium perchlorate, 80 ml of glacial acetic acid and 8.70 g (0.05 mole) of 4-[trifluoromethyl]--benzaldehyde is stirred on an oil bath of 150°C for 3 hours. Then the reaction mixture is worked up as follows. It is evaporated in vacuo. The residue is solidified in 100 ml of water, filtered, washed three times with 15 ml each of water, the crude product is suspended while hot in 400 ml of water for 30 minutes, filtered, washed three times with 15 ml each of water and dried at 80 to 100°C. Thus 13.30 g (58%) of the desired product decomposing at 197 to 201°C are obtained, which can be used for the further reactions without any purification.

### Example 10

### 1-[4'-(Cyano)-styryl]-3-[methyl]-6,7-[methylenedioxy]--2-benzopyrilium perchlorate

A mixture of 9.33 g (0.031 mole) of 1,3-di-[methyl]-6,7-- [methylenedioxy]-2-benzopyrilium perchlorate, 50 ml of glacial acetic acid and 4.0 g (0.032 mole) of 4-[cyano]-benzaldehyde is stirred on an oil bath of 150°C for 3 hours. Then it is worked up as specified in Example 1. Thus 9.30 g (75%) of the desired product decomposing at 241 to 247°C are obtained.

### Example 11

### 1-[3'-(Fluoro)-styryl]-3-[methyl]-6,7-[methylenedioxy]--2-benzopyrilium perchlorate

A mixture of 15.10 g (0.05 mole) of 1,3-di-[methyl]-6,7-- [methylenedioxy]-2-benzopyrilium perchlorate, 80 ml of glacial acetic acid and 6.20 g (0.05 mole) of 3-[fluoro]-benzaldehyde is stirred on an oil bath of 150°C for 3 hours. The mixture is then worked up as specified in Example 1. Thus 11.20 g (55%) of the desired product decomposing at 186 to 190°C are obtained.

### Example 12

### 1-[2'-(Fluoro)-styryl]-3-[methyl]-6,7-[methylenedioxy]--2-benzopyrilium perchlorate

A mixture of 15.10 g (0.05 mole) of 1,3-di-[methyl]-6,7--[methylenedioxy]-2-benzopyrilium perchlorate, 80 ml of glacial acetic acid and 6.20 g (0.05 mole) of 2-[fluoro]-benzaldehyde is stirred on an oil bath of 150°C for 3 hours. The reaction mixture is then worked up as specified in Example 1. Thus 10.0 g (49%) of the desired product decomposing at 235 to 238°C are obtained, which can be used for the further reactions without any purifications.

### Example 13

### 1-[4'-(Fluoro)-styryl]-3-[methyl]-7,8-[methylenedioxy]--2-benzopyrilium perchlorate

A mixture of 5.0 g (16.6 mmoles) of 1,3-di-[methyl]-7,8--[methylenedioxy]-2-benzopyrilium perchlorate, 30 ml of glacial acetic acid and 2.10 g (16.6 mmoles) of 4-[fluoro]-benzaldehyde is stirred on an oil bath of 150°C for 1 hour. The reaction mixture is then worked up as specified in Example 1. Thus 2.70 g (40%) of the desired compound decomposing at 205 to 209°C are obtained, which can be used for the further reactions without any purification.

## Claims

1. 1-[2'-(Substituted) vinyl]-5H-2,3-benzodiazepine derivatives of general formula 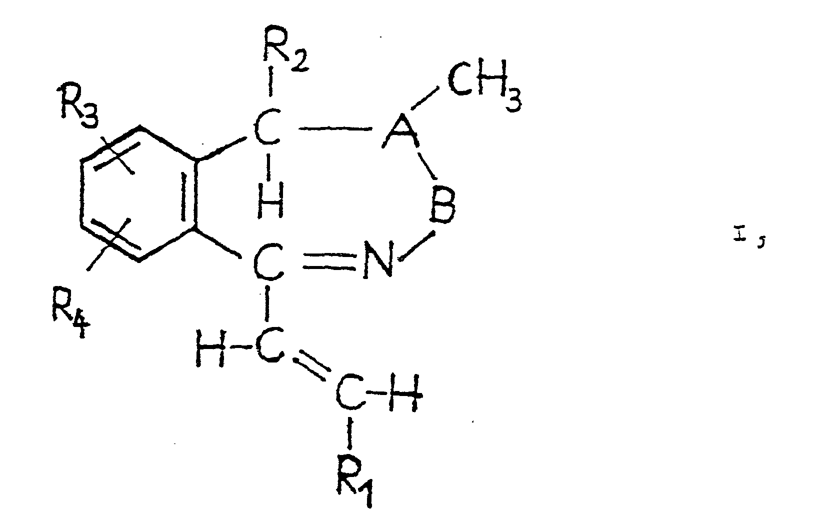 wherein
A and B together form a group of formula 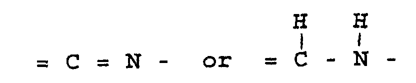
R₁ represents phenyl, carrying 1 to 3 identical or different substituent(s) selected from the group consisting of halogen, trifluoromethyl or cyano,
R₂ stands for hydrogen and
R₃ and R₄ together form a 7,8- or 8,9-methylenedioxy group
as well as acid addition salts and stereoisomers and mixtures thereof.

2. 1- [2' - (Substituted) vinyl] -5H-2,3-benzodiazepine derivatives according to claim 1, characterized in that the halogen atom(s) by which the phenyl group by which R₁ may be represented may be substituted is/are fluorine, bromine and/or chlorine.

3. 1-[2'-(Substituted) vinyl] -5H-2,3-benzodiazepine derivatives according to claim 1 or 2, characterized in that
A and B together form a group of formula
= C = N - ,
R₁ represents phenyl, carrying 1 or 2 fluoro, cyano and/or trifluoromethyl substituent(s),
R₂ stands for hydrogen and
R₃ and R₄ together form a 7,8-methylenedioxy group,
as well as acid addition salts and stereoisomers and mixtures thereof.

4. A compound according to claim 1, which is 1-[4'-(Fluoro)-styryl]-4-[methyl]-7,8-[methylenedioxy]-5H--2,3-benzodiazepine as well as acid addition salts and stereoisomers and mixtures thereof.

5. A process for preparing the 1-[2'-(substituted) vinyl]-5H--2,3-benzodiazepine derivatives according to claims 1 to 4, characterized in that
a) in a manner known per se for preparing 1-[2'--(substituted) vinyl]-5H-2,3-benzodiazepine derivatives of general formula I, wherein A and B together form a group of formula = C = N - , and R₁, R₂, R₃ and R₄ are as defined in claims 1 to 3, reacting 2-benzopyrilium perchlorate derivatives of general formula 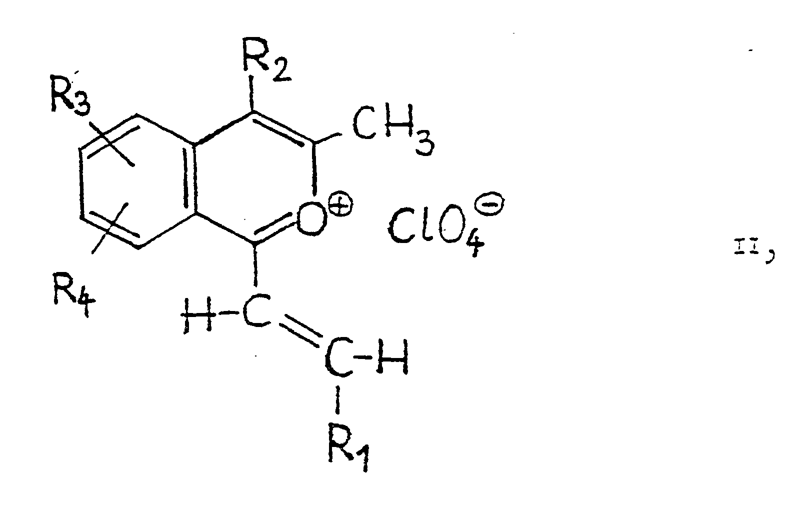 wherein R₁, R₂, R₃ and R₄ are as defined in claims 1 to 3, with hydrazine hydrate or
b) for preparing 1-[2'-(substituted) vinyl]-5H-2,3--benzodiazepine derivatives of general formula I, wherein A and B together form a group of formula 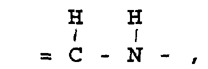 R₁ stands for phenyl carrying a fluoro atom, a trifluoromethyl or a cyano group, R₂ represents hydrogen and R₃ and R₄ together form a 7,8- or 8,9-methylenedioxy group, in a manner known per se reducing 1-[2'-(substituted) vinyl]-5H-2,3--benzodiazepine derivatives of general formula I, wherein A and B together form a group of formula = C = N - and R₁, R₂, R₃ and R₄ are as defined in claims 1 to 3, with complex metal hydrides, boranes and/or borane complexes, and optionally in a manner known per se converting the 1-[2'-(substituted) vinyl]-5H-2,3-benzodiazepine derivatives of general formula I obtained by any of the above variants a) or b) into acid addition salts thereof and/or optionally converting acid addition salts of 1-[2'-- (substituted) vinyl] -5H-2,3-benzodiazepine derivatives of general formula I obtained by any of the above variants a) to c) into the corresponding free 1-[2'-(substituted) vinyl]-5H-2,3-benzodiazepine derivatives of general formula I and/or into other acid addition salts and/or resolving stereoisomer mixtures of the 1-[2'--(substituted) vinyl]-5H-2,3-benzodiazepine derivatives of general formula I or acid addition salts thereof into their stereoisomers or converting the stereoisomers into mixtures thereof.

6. Medicaments, characterized in that they contain 1 or more 1-[2'-(substituted) vinyl]-5H-2,3-benzodiazepine derivative(s) according to claims 1 to 4 as [an] active ingredient(s), advantageously in admixture with 1 or more inert solid and/or liquid pharmaceutical carrier(s) and/or auxiliari(es).

7. Use of the 1-[2'-(substituted) vinyl]-5H-2,3-benzodiaze-pine derivatives according to claims 1 to 4, for preparing medicaments, particularly for the treatment of central nervous system disorders.

8. 2-Benzopyrilium perchlorate derivatives of general formula 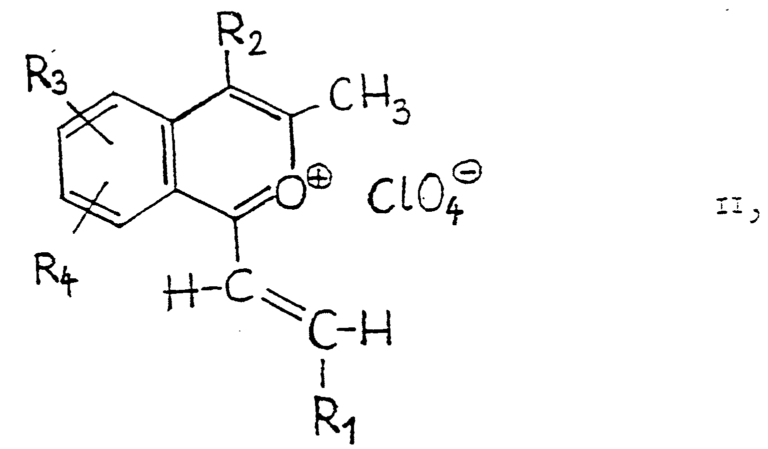 wherein
R₁ stands for phenyl carrying a fluoro atom, a trifluoromethyl or a cyano group,
R₂ represents hydrogen
and
R₃ and R₄ together form a 6,7- or 7,8-methylenedioxy group.

## Patentansprüche

1. 1-[2'-(substituiertes) Vinyl]-5H-2,3 -benzodiazepin-Derivate der allgemeinen Formel 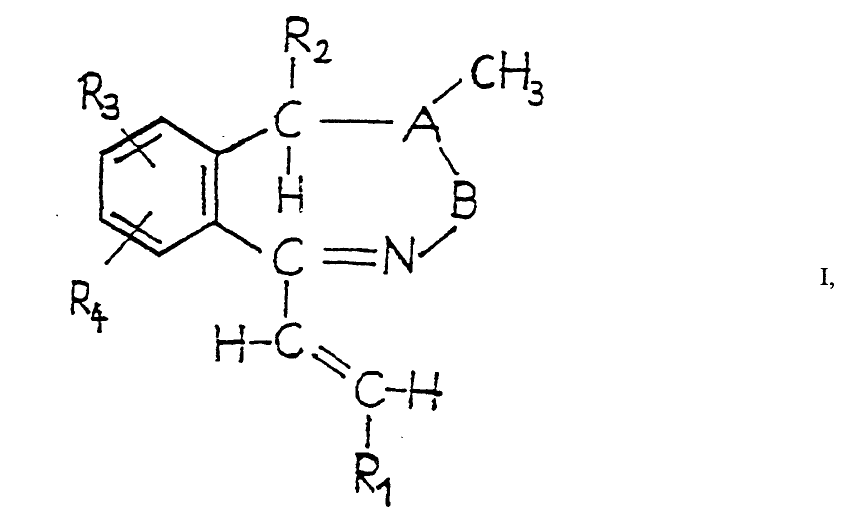 worin
A und B zusammen eine Gruppe der Formel 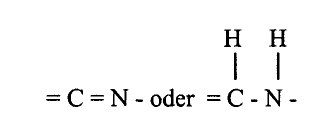 bilden,
R₁ Phenyl bedeutet, das 1 bis 3 identische oder unterschiedliche Substituenten trägt, gewählt aus der Gruppe bestehend aus Halogen, Trifluormethyl oder Cyano,
R₂ für Wasserstoff steht, und
R₃ und R₄ zusammen eine 7,8- oder 8,9-Methylendioxygruppe bilden,
sowie Säureadditionssalze und Stereoisomere und Mischungen davon.

2. 1-[2'-(substituiertes) Vinyl]-5H-2,3-benzodiazepin-Derivate gemäß Anspruch 1, dadurch gekennzeichnet, daß das (die) Halogenatom(e), durch welche(s) die Phenylgruppe, durch welche R₁ repräsentiert werden kann, substituiert werden kann, Fluor, Brom und/oder Chlor ist/sind.

3. 1-[2'-(substituiertes) Vinyl]-5H-2,3-benzodiazepin-Derivate gemäß Anspruch 1 oder 2, dadurch gekennzeichnet, daß
A und B zusammen eine Gruppe der Formel
= C = N -
bilden,
R₁ Phenyl bedeutet, das 1 oder 2 Fluor-, Cyano- und/oder Trifluormethyl-Substituenten trägt,
R₂ für Wasserstoff steht, und
R₃ und R₄ zusammen eine 7,8-Methylendioxygruppe bilden,
sowie Säureadditionssalze und Stereoisomere und Mischungen davon.

4. Verbindung gemäß Anspruch 1, welche 1-[4'-(Fluor)-styryl]-4-[methyl]-7,8-[methylendioxy]-5H-2,3-benzodiazepin sowie Säureadditionssalze und Stereoisomere und Mischungen davon ist.

5. Verfahren zur Herstellung der 1-[2'-(substituiertes) Vinyl]-5H-2,3-benzodiazepin-Derivate gemäß Anspruch 1 bis 4, gekennzeichnet durch
a) das Umsetzen in einer an sich bekannten Weise zur Herstellung von 1-[2'-(substituiertes) Vinyl]-5H-2,3-benzodiazepin-Derivaten der allgemeinen Formel I, worin A und B zusammen eine Gruppe der Formel = C = N - bilden und R₁, R₂, R₃ und R₄ wie in den Ansprüchen 1 bis 3 definiert sind, von 2-Benzopyriliumperchlorat-Derivaten der allgemeinen Formel 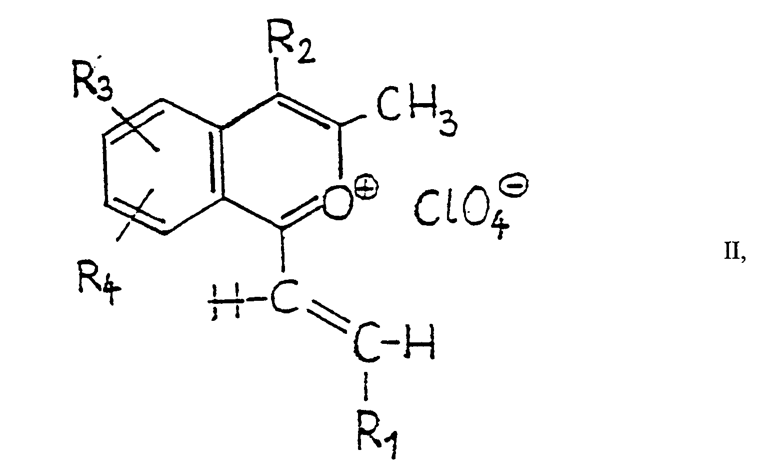 worin R₁, R₂, R₃ und R₄ wie in den Ansprüchen 1 bis 3 definiert sind, mit Hydrazinhydrat
oder
b) zur Herstellung von 1-[2'-(substituiertes) Vinyl]-5H-2,3-benzodiazepin-Derivaten der allgemeinen Formel I, worin A und B zusammen eine Gruppe der Formel 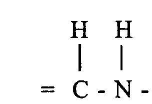 bilden, R₁ für Phenyl, das ein Fluoratom, eine Trifluormethyl- oder eine Cyanogruppe trägt, steht, R₂ Wasserstoff bedeutet und R₃ und R₄ zusammen eine 7,8- oder 8,9-Methylendioxygruppe bilden, das Reduzieren in einer an sich bekannten Weise von 1-[2'-(substituiertes) Vinyl]-5H-2,3-benzodiazepin-Derivaten der allgemeinen Formel I, worin A und B zusammen eine Gruppe der Formel = C = N- bilden und R₁, R₂, R₃ und R₄ wie in den Ansprüchen 1 bis 3 definiert sind, mit komplexen Metallhydriden, Boranen und/oder Borankomplexen,
und gegebenenfalls in einer an sich bekannten Weise das Umwandeln der 1-[2'-(substituiertes) Vinyl]-5H-2,3-benzodiazepin-Derivate der allgemeinen Formel I, erhalten durch eine der obenstehenden Varianten a) oder b), in Säureadditionssalze davon und/oder gegebenenfalls Umwandeln von Säureadditionssalzen von 1-[2'-(substituiertes) Vinyl]-5H-2,3-benzodiazepin-Derivaten der allgemeinen Formel I, erhalten durch eine der obenstehenden Varianten a) bis c), in die entsprechenden freien 1-[2'-(substituiertes) Vinyl]-5H 2,3-benzodiazepin-Derivate der allgemeinen Formel I und/oder in andere Säureadditionssalze und/oder Auftrennen von Stereoisomer-Mischungen der 1-[2'-(substituiertes) Vinyl]-5H-2,3-benzodiazepin-Derivate der allgemeinen Formel I oder Säureadditionssalze davon in ihre Stereoisomere oder Umwandeln der Stereoisomere in Mischungen davon.

6. Medikamente, dadurch gekennzeichnet, daß diese 1 oder mehr 1-[2'-(substituiertes) Vinyl]-5H-2,3-benzodiazepin-Derivate gemäß den Ansprüchen 1 bis 4 als (einen) aktive(n) Bestandteil(e) enthalten, vorteilhafterweise vermischt mit 1 oder mehr inerten festen und/oder flüssigen pharmazeutischen Träger(n) und/oder Hilfsstoff (Hilfsstoffe).

7. Verwendung der 1-[2'-(substituiertes) Vinyl]-5H-2,3-benzodiazepin-Derivate gemäß Anspruch 1 bis 4 zur Herstellung von Medikamenten, insbesondere zur Behandlung von Störungen des zentralen Nervensystems.

8. 2-Benzopyriliumperchlorat-Derivate der allgemeinen Formel 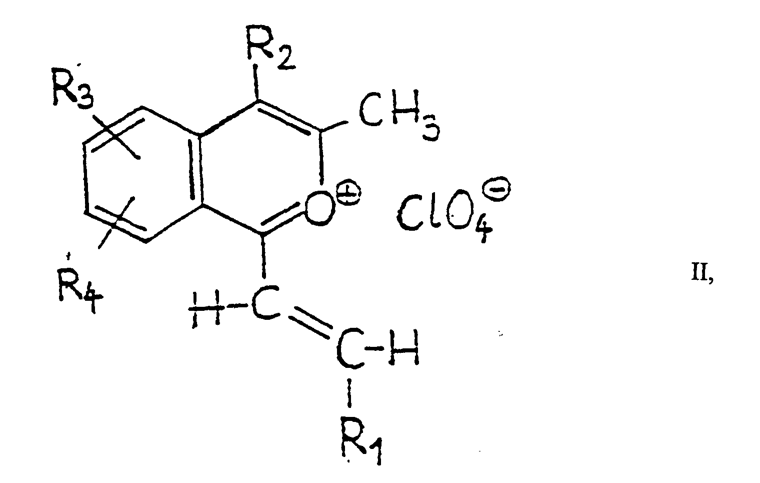 worin
R₁ für Phenyl, das ein Fluoratom, eine Trifluormethyl- oder Cyanogruppe trägt, steht,
R₂ Wasserstoff bedeutet,
und
R₃ und R₄ zusammen eine 6,7- oder 7,8-Methylendioxygruppe bilden.

## Revendications

1. Dérivés de 1-[2'-(substitué)-vinyl]-5H-2,3-benzodiazépine de formule générale : 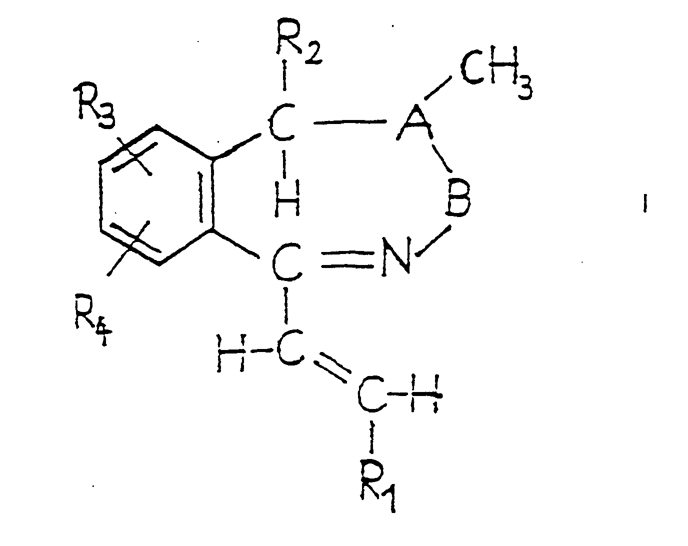 dans laquelle :
A et B forment ensemble un groupe de formule = C = N- ou = C(H) - N(H) -,
R₁ représente un groupe phényle, portant 1 à 3 substituants identiques ou différents choisis dans le groupe consistant en atome d'halogène, groupe trifluorométhyle ou cyano,
R₂ représente un atome d'hydrogène, et
R₃ et R₄ forment ensemble un groupe 7,8- ou 8,9- méthylènedioxy,
ainsi que des sels d'addition d'acide et stéréoisomères et mélanges de ceux-ci.

2. Dérivés de 1-[2'-(substitué)-vinyl]-5H-2,3-benzodiazépine suivant la revendication 1, caractérisés en ce que l'atome ou les atomes d'halogène par lesquels le groupe phényle représenté par R₁ peut être substitué, sont un ou des atomes de fluor, de brome et/ou de chlore.

3. Dérivés de 1-[2'-(substitué)-vinyl]-5H-2,3-benzodiazépine suivant les revendications 1 ou 2, caractérisés en ce que :
A et B forment ensemble un groupe de formule
= C = N - ,
R₁ représente un groupe phényle, portant 1 ou 2 atomes de fluor et/ou groupes trifluorométhyle ou cyano comme substituants,
R₂ représente un atome d'hydrogène, et
R₃ et R₄ forment ensemble un groupe 7,8-méthylènedioxy, ainsi que des sels d'addition d'acide et stéréoisomères et mélanges de ceux-ci.

4. Composé suivant la revendication 1, qui est la 1-[4'-(fluoro)-styryl]-4-[méthyl]-7,8- [méthylènedioxy]-5H-2,3-benzodiazépine, ainsi que des sels d'addition d'acide et stéréoisomères et mélanges de celle-ci.

5. Procédé pour la préparation des dérivés de 1-[2'-(substitué)-vinyl]-5H-2,3-benzodiazépine suivant les revendications 1 à 4, caractérisé en ce que :
a) selon une procédure connue en soi pour préparer des dérivés de 1-[2'-(substitué)-vinyl]-5H-2,3-benzodiazépine de formule générale I, dans laquelle A et B forment ensemble un groupe de formule = C = N -, et R₁, R₂, R₃ et R₄ sont tels que définis dans les revendications 1 à 3, on fait réagir des dérivés de type perchlorate de 2-benzopyrilium de formule générale : 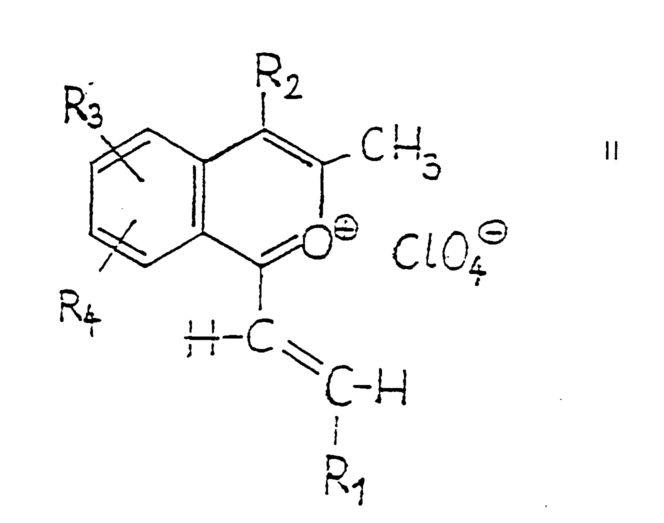 dans laquelle R₁, R₂, R₃ et R₄ sont tels que définis dans les revendications 1 à 3, avec de l'hydrazine hydratée, ou
b) pour préparer des dérivés de 1-[2'-(substitué)-vinyl]-5H-2,3-benzodiazépine de formule générale I, dans laquelle A et B forment ensemble un groupe de formule = C(H)-N(H) -, R₁ représente un groupe phényle portant un atome de fluor, un groupe trifluorométhyle ou cyano, R₂ est un atome d'hydrogène, et R₃ et R₄ forment ensemble un groupe 7,8- ou 8,9-méthylèedioxy, on réduit selon une procédure connue en soi des dérivés de 1-[2'-(substitué)-vinyl]-5H-2,3-benzodiazépine de formule générale I, dans laquelle A et B forment ensemble un groupe de formule = C = N -, et R₁, R₂, R₃ et R₄ sont tels que définis dans les revendications 1 à 3, avec des hydrures métalliques complexes, des boranes et/ou des complexes de borane, et éventuellement, selon une procédure connue en soi, on convertit les dérivés de 1-[2'-(substitué)-vinyl]-5H-2,3-benzodiazépine de formule générale I obtenus par l'une quelconque des variantes a) ou b) ci-dessus en leurs sels d'addition d'acide et/ou éventuellement on convertit les sels d'addition d'acide des dérivés de 1-[2'-(substitué)-vinyl]-5H-2,3-benzodiazépine de formule générale I obtenus par l'une quelconque des variantes a) ou b) ci-dessus en les dérivés libres correspondants de 1-[2'-(substitué)-vinyl]-5H-2,3-benzodiazépine de formule générale I et/ou en d'autres sels d'addition d'acide et/ou on procède à la résolution des mélanges stéréoisomères des dérivés de 1-[2'-(substitué)-vinyl]-5H-2,3-benzodiazépine de formule générale I ou de leurs sels d'addition d'acide en leurs stéréoisomères ou on convertit les stéréoisomères en leurs mélanges.

6. Médicaments, caractérisés en ce qu'ils contiennent un ou plusieurs dérivés de 1-[2'-(substitué)-vinyl]-5H-2,3-benzodiazépine suivant les revendications 1 à 4 en tant que composants actifs, avantageusement en mélange avec un ou plusieurs supports et/ou adjuvants pharmaceutiques inertes solides et/ou liquides.

7. Utilisation des dérivés de 1-[2'-(substitué)-vinyl]-5H-2,3-benzodiazépine suivant les revendications 1 à 4, pour la préparation de médicaments, en particulier pour le traitement de troubles du système nerveux central.

8. Dérivés de type perchlorate de 2-benzopyrilium de formule générale: 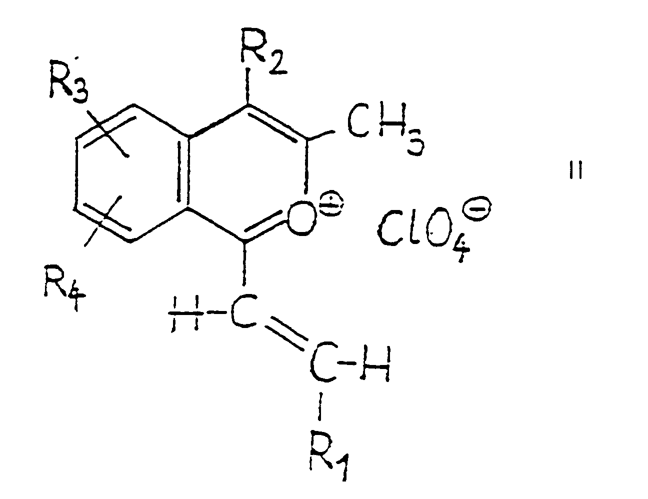 dans laquelle :
R₁ représente un groupe phényle, portant un atome de fluor, un groupe trifluorométhyle ou un groupe cyano,
R₂ représente un atome d'hydrogène, et R₃ et R₄ forment ensemble un groupe 6,7- ou 7,8- méthylènedioxy.
